# EUROPEAN PATENT SPECIFICATION

(11) **EP 3 547 861 B1**
(45) Date of publication and mention of the grant of the patent: **20.01.2021**
(21) Application number: 17829358.5
(22) Date of filing: 01.12.2017
(51) Int. Cl.: A24F 40/30, A24F 40/60

(54) **COMBINATION VAPORIZER**
KOMBINATIONSVERDAMPFER
VAPORISATEUR COMBINÉ

(30) Priority: 02.12.2016 US 201662429348 P; 01.03.2017 US 201762465419 P
(43) Date of publication of application: 09.10.2019
(73) Proprietor: VMR Products LLC, San Francisco, CA 94107 (US)
(72) Inventor: VERLEUR, Jan, Andries, San Francisco, CA 94107 (US); RECIO, Dan, San Francisco, CA 94107 (US); LIU, Zhiyuan, San Francisco, CA 94107 (US); VERLEUR, Hans, San Francisco, CA 94107 (US)
(74) Representative: Thum, Bernhard
(86) International application number: PCT/US2017/064244
(87) International publication number: WO 2018/102703

(56) References cited:
- WO-A1-2014/110750
- WO-A1-2015/128499
- WO-A1-2016/090426
- WO-A1-2017/174754
- US-A1- 2012 048 266
- US-A1- 2015 196 059

## Description

### CROSS REFERENCE TO RELATED APPLICATIONS

This application claims the benefit of U.S. Provisional Application Serial Number 62/429,348, filed December 2, 2016 and U.S. Provisional Application Serial Number 62/465,419, filed March 1, 2017.

### BACKGROUND

### 1. Technical Field text

This disclosure relates generally to vaporizers, which may also be referred to as electronic cigarettes.

### 2. Background Information

Vaporizers have recently emerged as a new product for providing nicotine and other products through a smokeless inhalation process. There are many embodiments of vaporizers including the electronic cigarette. In general, implementations consist of a power supply (typically a battery) and an atomizing device. In reusable electronic cigarettes, the two items are separated into a battery and a cartomizer, to allow the disposal and replacement of the nicotine containing fluid cartomizer while preserving for additional use the more costly battery and associated circuitry (microcontroller, switch, indicating light emitting diode (LED), etc.). In disposable electronic cigarettes the two functions are integrated into one unit that is disposed of after exhaustion of either the battery energy or the vaporizable fluid ("E- liquid"), typically nicotine-containing, that is associated with the cartomizer.

The E-liquid that is used to produce vapor in electronic cigarettes is typically a flavor concentrate, optionally with a variable percentage of a liquid nicotine concentrate, dissolved in a solution of one or more of propylene glycol (PG) and/or vegetable glycerin (VG) and/or polyethylene glycol 400 (PEG400). This liquid is often sold in a bottle or in disposable cartridges or cartomizers. Many different flavors of such E-liquids are sold, including flavors that resemble the taste of regular tobacco, menthol, vanilla, coffee, cola and various fruits. E-liquids containing a wide range of nicotine concentrations, as well as nicotine-free liquids are available in the marketplace.

In addition to E-liquid, other products may be used to produce vapor such as waxes and solids such as loose leafs. Generally, each type of product requires a specific type of atomizer. A loose leaf product may be vaporized by a hot stream of gas, whereas waxes and E-liquids may be vaporized upon contact with a heated element.

WO 2015/128499 A1 describes a smoking device having a housing, a liquid evaporator, which is arranged in the housing, and a reservoir for liquid and a liquid-heating device for evaporating the liquid into liquid vapour, and a tobacco heater, which is arranged in the housing and has a heating chamber for accommodating a tobacco substance in a chamber interior and a chamber heating device for generating tobacco vapour from the tobacco substance, and an intake-opening arrangement for taking in the liquid vapour and for taking in the tobacco vapour from the housing, wherein the liquid vapour flows, in a liquid-flow channel, from the liquid-heating device to the intake-opening arrangement, and the tobacco vapour flows, in a tobacco-flow channel, from the chamber interior to the intake-opening arrangement.

### BRIEF SUMMARY

A combination vaporizer and a method for delivering a combination of vapor from two vaporizable products according to the invention are defined in the claims. A vaporizer for vaporizing combinations of product useful for understanding the invention is disclosed in the following. The vaporizer includes a first a first body, a cartridge, a second body, a controller, and a fluid path. The first body has an air inlet and a cavity for receiving the cartridge. The cartridge is disposed in the cavity and contains a first product. A second body contains a second product and has a recess complementary to the shape of the first body. The controller is in communication with the first cartridge and the second body and is configured to control the amount of vapor produced by each cartridge. The fluid path passes from the second body through the inlet to the first body to provide vapor from the second body to the first cartridge.

### BRIEF DESCRIPTION OF THE DRAWINGS

FIG. 1 illustrates a perspective view of a vaporizer.
FIG. 2 illustrates an exploded view of the vaporizer of FIG. 1.
FIG. 3 illustrates cartridges for use in the vaporizer of FIG. 1.
FIG. 4 illustrates a schematic view of the airflow of the vaporizer of FIG. 1.
FIG. 5 illustrates an embodiment of an add-on tank.
FIG. 6 illustrates a schematic view of the airflow of the add-on tank of FIG. 5.
FIG. 7 illustrates an embodiment of a vaporizer combined with an add-on tank.
FIG. 8 illustrates a schematic view of the airflow of the embodiment of FIG. 7.
FIG. 9 illustrates an embodiment of a method for producing custom vapor.

### DETAILED DESCRIPTION

The following detailed description and the appended drawings describe and illustrate some embodiments of the disclosure for the purpose of enabling one of ordinary skill in the relevant art to make and use these embodiments. As such, the detailed description and illustration of these embodiments are purely illustrative in nature and are in no way intended to limit the scope of the disclosure in any manner. It should also be understood that the drawings are not necessarily to scale and in certain instances details may have been omitted, which are not necessary for an understanding of the embodiments, such as details of fabrication and assembly. In the accompanying drawings, like numerals represent like components.

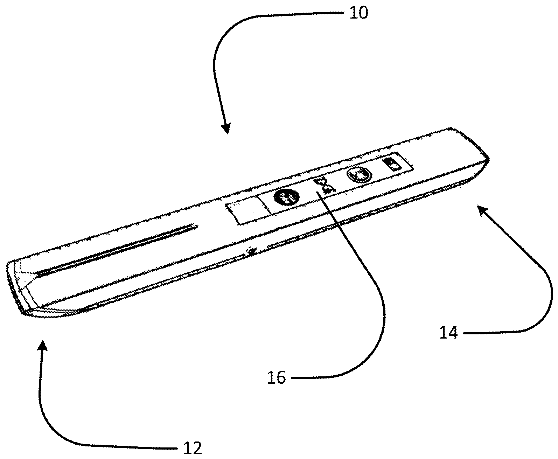

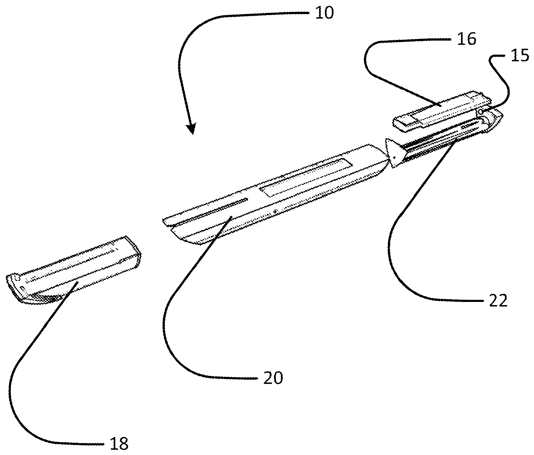

FIG. 1 illustrates a perspective view of a vaporizer 10. Vaporizer 10 receives product, such as E-liquid, wax, or solids, in cartridges that are loaded into vaporizer 10. Because each type of product may require a different type of atomizer, each cartridge may have an integral atomizer. One of ordinary skill in the art will recognize that various types of atomizers currently exist and may be used to vaporize product in a cartridge. Vaporizer 10 supplies power to the cartridge, which activates the atomizer to produce vapor from the product. The vapor is in turn mixed with incoming air for delivery to a user. Vaporizer 10 typically contains a power source such as a battery, and a control panel 16 providing an interface for a user to operate vaporizer 10. A lower end 14 of vaporizer 10 has an inlet 15 (FIG. 2) for receiving incoming air and an upper end 12 of vaporizer 10 may have an outlet for delivering vapor. Inlet 15 may be in addition to a cartridge inlet for receiving air, or it may be the only inlet. Inlet 15 provides a fluid path through the vaporizer to the cartridge. The vaporizer 10 may also have a power port for charging the battery, or an inductive element for wirelessly charging the battery.

FIG. 2 illustrates an exploded view of first vaporizer element 10. The vaporizer 10 includes cartridge 18, body 20, control panel 16, and battery 22. Battery 22 and control panel 16 may be permanently secured within body 20, while cartridge 18 is removable from body 20. Control panel 16 is electrically coupled to battery 22 and cartridge 18 when assembled.

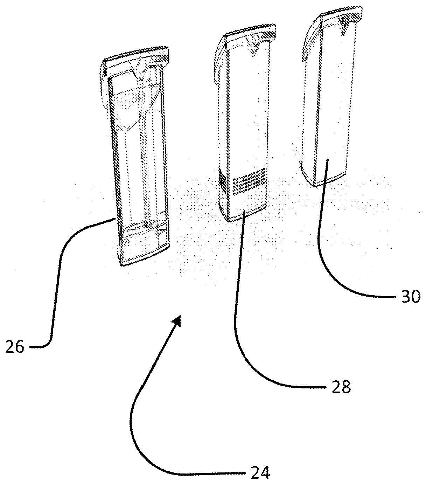

FIG. 3 illustrates cartridges 24 for use in first vaporizer element 10. Cartridges 24 include loose leaf cartridge 26, wax cartridge 28, and E-liquid cartridge 30. Cartridges 24 may be releasably secured within the upper end 12 of the vaporizer 10 and exchanged with one another depending on user preference. Each cartridge 24 contains product in a vaporizable product container 11 and an atomizer 13 specific to that product type. Cartridge 24 may be refillable by a user. Control panel 16 may recognize the type of cartridge 24 in use by the vaporizer 10 and adjust vaporizing parameters such as voltage delivered to the cartridge and timing as necessary for the product type. The control panel 16 may allow further customization by the user or a manual setup for product not recognized by the control panel 16.

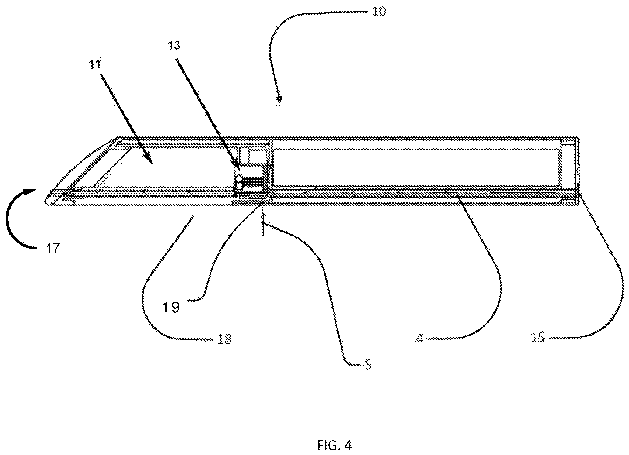

FIG. 4 illustrates a schematic view of the vaporizer 10 of FIG. 1. Arrow 4 illustrates an air path from the lower end of the vaporizer 10 from inlet 15. Arrow 5 illustrates an alternative air path using inlet 19. Embodiments may have a selectable air path, such that air may flow along arrow 4 or arrow 5. Vaporizable liquid, after vaporization in the atomizer 13, is exhausted to the external environment via vapor outlet 17.

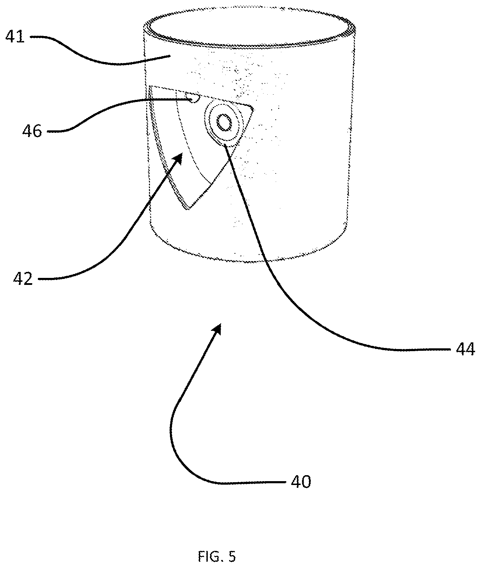

FIG. 5 illustrates an add-on tank 40 (second vaporizer element) which enhances vaporizer 10. Add-on tank 40 is configured to couple to vaporizer 10 and provide enhancements such as extra battery life, an additional heating unit, and/or additional product. In the embodiment of FIG. 4, add-on tank 40 has a body 41 with a recess 42 that complements the shape of lower end 14 of vaporizer 10. Lower end 14 of vaporizer 10 may be inserted into recess 42 to couple vaporizer 10 and add-on tank 40 together. While add-on tank 40 is shown with a cylindrical body, other shapes are possible such as a box or sphere.

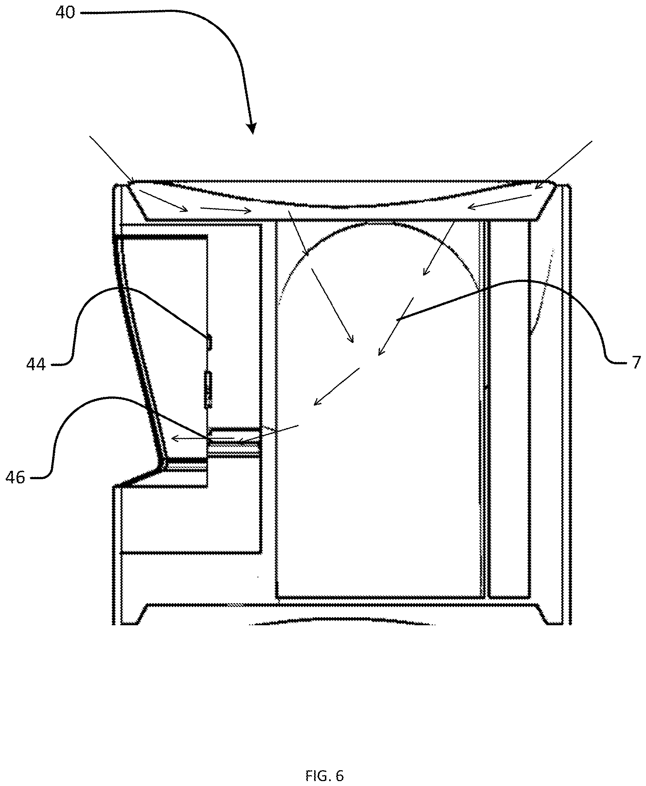

FIG. 6 illustrates a schematic view of add-on tank 40 of FIG. 5. Add-on tank 40 has a flow path depicted by arrows 7. Add-on tank 40 includes an interface 44 for communicating with vaporizer 10. Interface 44 may interface with vaporizer 10 through a charging port, or in outer embodiment may have electrical contacts that contact corresponding contacts of the vaporizer 10. Control panel 16 may identify the add-on tank 40 and modify the interface accordingly. Add-on tank 40 further includes a port 46 that aligns with air inlet 15 of vaporizer 10 when connected. Port 46 allows vaporizer 10 to continue to receive air when add-on tank 40 is secured to vaporizer 10.

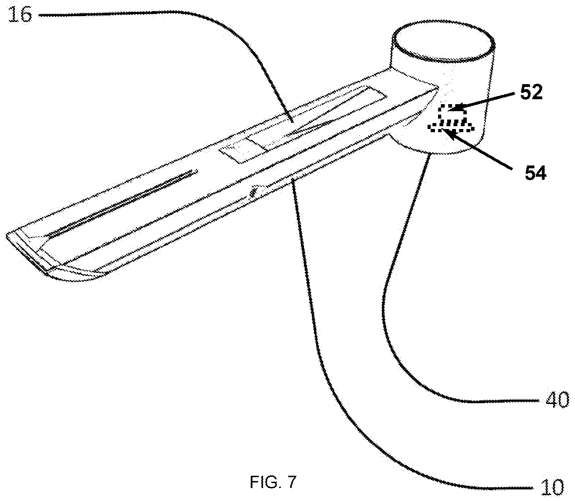

FIG. 7 illustrates vaporizer 10 being secured to add-on tank 40. In some embodiments, vaporizer 10 and add-on tank 40 may be secured to one another using magnets contained in lower end 14 of vaporizer 10 and in add-on tank 40. FIG. 7 also illustrates an atomizer 52 and a heating element 54, each hidden from view. Intake air passes through port 46 or vapor outlet 56.

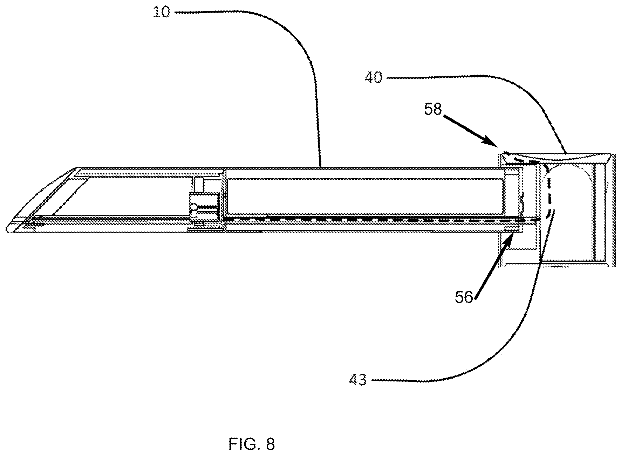

FIG. 8 illustrates a schematic of vaporizer 10 being connected to add-on tank 40. Flow path 43 passes through add-on tank 40 and into vaporizer 10. In embodiments in which add-on tank 40 is a battery, flow path 43 may be a simple channel passing through the add-on tank 40 for providing air to inlet 15 of vaporizer 10. In other embodiments, add-on tank 40 may contain an additional heater and additional product, including a product container, for vaporizing. In such embodiments, vapor from add-on tank 40 and air is supplied to vaporizer 10 through port 46.

The additional atomizer in add-on tank 40 allows for the vaporizer to deliver combinations of vapor from separate products. Control panel 16 may control the relative amount of vaporization between the two atomizers, which may be adjustable based on user preference. The add-on tank 40 allows for combinations of product such as E-liquid and loose leaf, E-liquid and wax, E-liquid (flavor 1) and E-liquid (flavor 2), and Loose Leaf and Wax. In addition to these combinations, the user may adjust the relative quantities of the product to obtain a diversity of product.

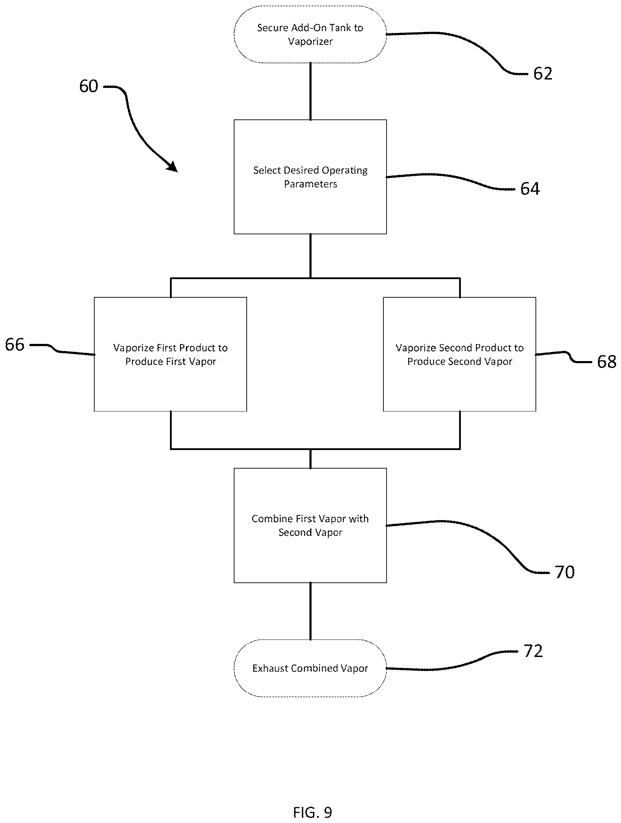

FIG. 9 illustrates a method 60 for delivering a combination of vapor from different products. At step 62 an add-on tank is secured to a vaporizer, such as add-on tank 40 to vaporizer 10. The desired operating parameters are selected at step 64. This may be done through control panel 14. A first product is vaporized at step 66 and a second product is vaporized at step 68. For example, a first product may be vaporized in cartridge 24 and a second product may be vaporized in add on tank 40. The two vapors are then combined in step 70. The two vapors may be combined by channeling second vapor through port 46 and through vaporizer 10. The second vapor then enters cartridge 24 to mix with first vapor. The combined vapor is then exhausted in step 72. The combined vapor may be exhausted through outlet of vaporizer 10.

The descriptions set forth above are meant to be illustrative and not limiting.

The foregoing description of possible implementations consistent with the present disclosure does not represent a comprehensive list of all such implementations or all variations of the implementations described. The description of some implementation should not be construed as an intent to exclude other implementations. For example, artisans will understand how to implement the invention in many other ways, using equivalents and alternatives that do not depart from the scope of the invention. Moreover, unless indicated to the contrary in the preceding description, none of the components described in the implementations are essential to the invention. It is thus intended that the embodiments disclosed in the specification be considered as illustrative, with a true scope of the invention being indicated by the following claims.

## Claims

1. A combination vaporizer, comprising:
a first vaporizer element (10) comprising a first body (20) having an upper end (12) and a lower end (14), the first body having a cavity in the upper end for receiving a cartridge (18, 24), a first air inlet (15, 19), and a first vapor outlet (17),
a first cartridge (18, 24) disposed in the cavity, the first cartridge containing a first product and a first atomizer (13) for atomizing the first product, and
a battery (22) secured in the first body;
a second vaporizer element (40) comprising a second body (41) containing a heating element (54), a second air inlet, a second vapor outlet (46, 56), and a second product, the second body having a recess (42) complementary to a shape of the lower end (14) of the first body thereby allowing the lower end of the first vaporizer element to be inserted into the recess;
a controller (16) in electrical communication with the first cartridge (18, 24) and the second vaporizer element (40), the controller configured to control an amount of vapor produced by the first cartridge (18, 24) and the second body (41);
a first fluid path passing from the second vapor outlet through the first air inlet to the first cartridge for combining vapor from the second vaporizer element with the vapor produced by the first cartridge; and
a second fluid path passing from the first cartridge to the first vapor outlet.

2. A vaporizer according to Claim 1, wherein the first cartridge (18, 24) is removable from the first body.

3. The vaporizer according to Claim 1, wherein fluid and electrical connection between the first body (20) and second body (41) are established when the lower end of the first body is inserted into the recess of the second body.

4. The vaporizer according to Claim 1, wherein the first product is a material comprising any one of a solid material, a wax material, and a liquid.

5. The vaporizer according to Claim 1, wherein the controller is configured to identify the second vaporizer element (40).

6. The vaporizer according to Claim 1, wherein the first product comprises a first material, and the second product comprises a second material differing from the first material.

7. The vaporizer according to Claim 1, wherein the controller is configured to recognize a type of the first cartridge and to adjust a voltage delivered to the cartridge.

8. The vaporizer according to Claim 1, wherein the second vapor outlet (46, 56) is configured to align with the first vapor inlet.

9. The vaporizer according to Claim 1, wherein the first vaporizer element (10) further comprises a power port for charging the battery (22).

10. The vaporizer according to Claim 1, wherein the first vaporizer element (10) further comprises an inductive element for wirelessly charging the battery.

11. A method for delivering a combination of vapor from a first vaporizable product and a second vaporizable product, said method comprising:
providing a combination vaporizer according to Claim 1;
securing the second vaporizer element to the first vaporizer element;
selecting operating parameters for the first atomizer and the heating element;
vaporizing the first product;
vaporizing the second product;
combining the vaporized first product with the vaporized second product; and
removing the combined vapor from the vaporizer.

## Patentansprüche

1. Kombinationsverdampfer, umfassend:
ein erstes Verdampferelement (10), das einen ersten Körper (20) mit einem oberen Ende (12) und einem unteren Ende (14) umfasst, wobei der erste Körper im oberen Ende einen Hohlraum zum Aufnehmen einer Kartusche (18, 24), einen ersten Lufteinlass (15, 19) und einen ersten Dampfauslass (17) aufweist,
eine erste Kartusche (18, 24), die in dem Hohlraum angeordnet ist, wobei die erste Kartusche ein erstes Produkt und einen ersten Zerstäuber (13) zum Zerstäuben des ersten Produkts enthält, und
eine Batterie (22), die in dem ersten Körper gesichert ist;
ein zweites Verdampferelement (40) das einen zweiten Körper (41) mit einem Heizelement (54), einem zweiten Lufteinlass, einem zweiten Dampfauslass (46, 56) und einem zweiten Produkt, umfasst, wobei der zweite Körper eine Aussparung (42) aufweist, die zu einer Form des unteren Endes (14) des ersten Körpers komplementär ist, wodurch das untere Ende des ersten Verdampferelements in die Aussparung eingesetzt werden kann;
einen Controller (16) der mit der ersten Kartusche (18, 24) und dem zweiten Verdampferelement (40) in elektrischer Verbindung steht, wobei der Controller dazu eingerichtet ist, eine von der ersten Kartusche (18, 24) und dem zweiten Körper (41) erzeugte Dampfmenge zu regeln;
einen ersten Fluidpfad, der von dem zweiten Dampfauslass durch den ersten Lufteinlass zu der ersten Kartusche verläuft, um Dampf von dem zweiten Verdampferelement mit dem von der ersten Kartusche erzeugten Dampf zu kombinieren; und
einen zweiten Fluidpfad, der von der ersten Kartusche zum ersten Dampfauslass führt.

2. Verdampfer nach Anspruch 1, wobei die erste Kartusche (18, 24) von dem ersten Körper entfernbar ist.

3. Verdampfer nach Anspruch 1, wobei fluidische und elektrische Verbindungen zwischen dem ersten Körper (20) und dem zweiten Körper (41) hergestellt werden, wenn das untere Ende des ersten Körpers in die Aussparung des zweiten Körpers eingeführt wird.

4. Verdampfer nach Anspruch 1, wobei das erste Produkt ein Material ist, das entweder ein festes Material, ein Wachsmaterial oder eine Flüssigkeit umfasst.

5. Verdampfer nach Anspruch 1, wobei der Controller dazu eingerichtet ist, das zweite Verdampferelement (40) zu identifizieren.

6. Verdampfer nach Anspruch 1, wobei das erste Produkt ein erstes Material umfasst und das zweite Produkt ein zweites Material umfasst, das sich von dem ersten Material unterscheidet.

7. Verdampfer nach Anspruch 1, wobei der Controller dazu eingerichtet ist, einen Typ der ersten Kartusche zu erkennen und eine an die Kartusche geleitete Spannung einzustellen.

8. Verdampfer nach Anspruch 1, wobei der zweite Dampfauslass (46, 56) so eingerichtet ist, dass er mit dem ersten Dampfeinlass ausgerichtet ist.

9. Verdampfer nach Anspruch 1, wobei das erste Verdampferelement (10) des Weiteren einen Stromanschluss zum Laden der Batterie (22) umfasst.

10. Verdampfer nach Anspruch 1, wobei das erste Verdampferelement (10) des Weiteren ein induktives Element zum drahtlosen Laden der Batterie umfasst.

11. Verfahren zum Zuführen einer Kombination von Dampf von einem ersten verdampfbaren Produkt und einem zweiten verdampfbaren Produkt, wobei das Verfahren umfasst:
Bereitstellen eines Kombinationsverdampfers nach Anspruch 1;
Befestigen des zweiten Verdampferelements an dem ersten Verdampferelement;
Selektives Auswählen von Betriebsparametern für den ersten Zerstäuber und das Heizelement;
Verdampfen des ersten Produkts;
Verdampfen des zweiten Produkts;
Kombinieren des verdampften ersten Produkts mit dem verdampften zweiten Produkt;
und
Entfernen des kombinierten Dampfs aus dem Verdampfer.

## Revendications

1. Vaporisateur combiné, comprenant :
un premier élément de vaporisateur (10) comprenant un premier corps (20) ayant une extrémité supérieure (12) et une extrémité inférieure (14), le premier corps ayant une cavité dans l'extrémité supérieure pour recevoir une cartouche (18, 24), une première entrée d'air (15, 19) et une première sortie de vapeur (17),
une première cartouche (18, 24) disposée dans la cavité, la première cartouche contenant un premier produit et un premier atomiseur (13) pour atomiser le premier produit, et
une batterie (22) fixée dans le premier corps ;
un deuxième élément de vaporisateur (40) comprenant un deuxième corps (41) contenant un élément chauffant (54), une deuxième entrée d'air, une deuxième sortie de vapeur (46, 56) et un deuxième produit, le deuxième corps ayant un évidement (42) complémentaire d'une forme de l'extrémité inférieure (14) du premier corps permettant ainsi à l'extrémité inférieure du premier élément de vaporisateur d'être insérée dans l'évidement ;
un contrôleur (16) en communication électrique avec la première cartouche (18, 24) et le deuxième élément de vaporisateur (40), le contrôleur étant configuré pour réguler une quantité de vapeur produite par la première cartouche (18, 24) et le deuxième corps (41) ;
un premier trajet de fluide passant de la deuxième sortie de vapeur à travers la première entrée d'air à la première cartouche pour combiner la vapeur provenant du deuxième élément de vaporisateur avec la vapeur produite par la première cartouche ; et
un deuxième trajet de fluide passant de la première cartouche à la première sortie de vapeur.

2. Vaporisateur selon la revendication 1, dans lequel la première cartouche (18, 24) est amovible du premier corps.

3. Vaporisateur selon la revendication 1, dans lequel la connexion fluidique et électrique entre le premier corps (20) et le deuxième corps (41) est établie lorsque l'extrémité inférieure du premier corps est insérée dans l'évidement du deuxième corps.

4. Vaporisateur selon la revendication 1, dans lequel le premier produit est un matériau comprenant l'un quelconque parmi un matériau solide, un matériau de cire et un liquide.

5. Vaporisateur selon la revendication 1, dans lequel le contrôleur est configuré pour identifier le deuxième élément de vaporisateur (40).

6. Vaporisateur selon la revendication 1, dans lequel le premier produit comprend un premier matériau, et le deuxième produit comprend un deuxième matériau différent du premier matériau.

7. Vaporisateur selon la revendication 1, dans lequel le contrôleur est configuré pour reconnaître un type de la première cartouche et pour régler une tension délivrée à la cartouche.

8. Vaporisateur selon la revendication 1, dans lequel la deuxième sortie de vapeur (46, 56) est conçue pour s'aligner avec la première entrée de vapeur.

9. Vaporisateur selon la revendication 1, dans lequel le premier élément de vaporisateur (10) comprend en outre un port d'alimentation pour charger la batterie (22).

10. Vaporisateur selon la revendication 1, dans lequel le premier élément de vaporisateur (10) comprend en outre un élément inductif pour charger sans fil la batterie.

11. Procédé pour distribuer une combinaison de vapeur à partir d'un premier produit vaporisable et d'un deuxième produit vaporisable, ledit procédé comprenant :
fournir un vaporisateur combiné selon la revendication 1 ;
fixer le deuxième élément de vaporisateur au premier élément de vaporisateur ;
sélectionner des paramètres de fonctionnement pour le premier atomiseur et l'élément chauffant ;
vaporiser le premier produit ;
vaporiser le deuxième produit ;
combiner le premier produit vaporisé avec le deuxième produit vaporisé ; et
retirer la vapeur combinée du vaporisateur.
